# EUROPEAN PATENT APPLICATION

(11) **EP 0 694 560 A2**
(43) Date of publication of application: **31.01.1996**
(21) Application number: 95305250.3
(22) Date of filing: 27.07.1995
(51) Int. Cl.: C07K 14/285, C07K 16/12, A61K 39/102, C12P 21/08, G01N 33/68

(54) **Production of antigens of Pasteurella**

(30) Priority: 29.07.1994 US 282702
(71) Applicant: AMERICAN CYANAMID COMPANY, Wayne, NJ 07470-8426 (US)
(72) Inventor: Rice Conlon, Jennifer Anne, Fort Dodge, Iowa 50501 (US); Walker, John A., Balwyn, Victoria 3103 (AU)
(74) Representative: Walters, Philip Bernard William

(57) **Abstract**

The present invention relates to generation of specific antigens associated with P. multocida under controlled culture conditions and the use of such conditions in monitoring the generation of specific antigens.

The invention relates to antigens preferably derived from culture supernatant, and most preferably the invention relates to identifying and purifying antigens from culture supernatant of a culture of P. multocida.

The present invenltion also relates to these antigens and the use of such antigens in the detection and purification of a number of protective and diagnostic antigens and antibodies from parasites and particularly bacteria. In particular, the present invention relates to the identification and purification of antigens in the bacterium family Pasteurellae, preferably Pasteurella multocida, and most preferably Pasteurella multocida type A:3.

The invention also relates to the use of the antigens in vaccines and in assays for diagnostic purposes.

The vaccines of the invention have antigens produced in accordance with the invention, preferably the vaccines have antigens derived from a culture supernatant of a culture of bacterium growth under controlled conditions, and more preferably, growth under iron-restricted culture conditions.

## Description

### BACKGROUND OF THE INVENTION

Considerable effort has been devoted in the prior art to the development of vaccines to control parasitic, bacterial and other infections of animals including livestock. However, little progress has been achieved to this end in the past 5 years although the associated technology of producing foreign products in large amounts in eukaryotic and prokaryotic organisms has advanced enormously. The identification of antigens in important pathogens that infect animals, for example, has remained the principal stumbling block to the generation of vaccines.

Bacterins incorporating infectious microorganisms have been used to prevent disease with variable success. Evidence from field studies and experimental trials suggests an adverse effect from vaccination using bacterins where only surface antigens are presented. Animals vaccinated with inactivated whole cell bacterins frequently show a higher incidence of disease and more severe lesions at post mortem than do unvaccinated animals.

Pneumonic pasteurellosis is an important widespread disease of both wild and domesticated animals. The disease is often due to an interaction between environmental factors and microbial agents such as Pasteurella multocida and Pasteurella haemolytica. In the raising of animals for commercial purposes, these organisms can be a significant cause of animal death. In cattle, "Shipping Fever" pneumonia is the major cause of sickness and mortality in feedlot cattle. (Martin et al. 1980)
Although several respiratory viruses and bacteria have been implicated in the pathogenesis of Shipping Fever the Pasteurellae have been reported as being the causative agent. These gram-negative non-motile, fermentative, faculatively anaerobic coccobacilli or rods are primarily animal pathogens adapted to parastic life on the oral and upper respiratory epithelia of apparently healthy animals and occasionally humans. (Allen et al, 1991)
Infection usually occurs as a consequence of stress such as overcrowding, chilling, transportation or intercurrent infection. Hence, the pasteurelloses are of major pathologic and economic significance in veterinary medicine.

Pasteurella haemolytica has been cultured to release a leukotoxin which has been used in a vaccine for anti-leukotoxic immunity to pneumonic pasteurellosis (US 5165924). However, unlike P. haemolytica, P. multocida does not produce leukotoxin. Very little is known about the virulence factors of this organism.

Pasteurella multocida has been extensively investigated as the cause of fowl cholera and subsequently identified in association with rabbit septicemia, swine plague, bovine pneumonia and hemorrhagic septicemia. Growth on artificial media is best achieved with the addition of serum or blood and is distinguished over other main species such as Pasteurella haemolytica, by lack of hemolysis on blood agar and failure to grow on MacConkey's agar.

Five serogroups and 16 somatic serotypes have been identified to differentiate the organism. The capsular serogroups are typically identified by a hemagglutinin test while the somatic antigens may be identified by a gel diffusion precipitate test. The five serotypes are based on capsular polysaccharide antigens and are outlined in Table 1.

**TABLE 1**

| Characteristics of strains of Pasteurella multocida Types A-F | |
|---|---|
| Type | Characteristics |
| A | Part of the normal flora of the respiratory tract of many domestic animals; associated with disease, usually respiratory, in stressed animals; includes most isolates from fowl cholera. |
| B | Noncommensal; associated with hemorrhagic septicemia of ruminants in Asia and Australia. |
| C | Part of the normal flora of dogs and cats; not encapsulated and thus not a valid type. |
| D | Like A, part of the normal flora; causes disease in stressed animals; includes strains exclusively associated with atrophic rhinitis of pigs. |
| E | Noncommensal; causes hemorrhagic septicemia in ruminants in central Africa. |
| F | Associated with disease in turkeys. |

Pasturella multocida type A and occasionally D are commonly implicated in bacterial pneumonia in a number of animals including dogs, cats, goats, sheep and cattle with several somatic serotypes being isolated in each species. Pneumonic pasteurellosis in feedlot cattle is typically due to type A³ (capsular group A, somatic antigen 3) although type A⁷ may also be present. The disease is characterized pathologically by a suppurative bronchitis and bronchopneumonia with little or no exudation of fibrin.

This disease may be reproduced experimentally by innoculation of the microorganism, eg. intra trachael innoculation.

Vaccines comprising whole P. multocida either live (Chengappa et al (1989)) or heat killed (Wijewardana and Sutherland (1990)) have been tested to show that a type of protection against further infection by P. multocida can be obtained with these whole vaccines. However, these vaccines have had little impact on disease control of this organism.

In the live vaccines, it was postulated that enhanced protective effects were due to protection of bacterial antigens. Killing organisms for the purposes of obtaining bacterins may result in loss of quantity and type, of possibly important antigens. However, the antigens involved in the enhanced protective effects have never been identified.

Effective live vaccines may provide a source of intact and possibly protective antigen useful in a more efficacious, single antigen vaccine, however, the use of live vaccines is difficult in the livestock industry since prophylactic antibiotic therapy is commonly used and the viability of a live vaccine would be in question.

Whole cell vaccines also introduce potentially harmful extraneous antigens into a vaccine preparation. Single antigen vaccines, or vaccines having the contents known greatly enhances the ability to regulate the immune response.

The ability of P. multocida to acquire iron from bovine transferrin in vivo has been demonstrated. (Ogunnariwo et al, 1991).

Iron Regulating Proteins (IRPS) have been described for serotypes of P. haemolytica and antibodies against these proteins have been found in immune-recovered lambs vaccinated with IRPS induced under iron-regulating conditions (Gilmore et al, 1991).

Little is known about iron acquisitation during P. multocida infection, yet P. multocida cell death due to antibody and complement was prevented by availability of iron for bacterial growth. Also, experimental infection with P. multocida in rabbits resulted in the reduction of plasma iron concentration plus fever as part of a coordinated host defence against pasteurellosis.

### SUMMARY OF THE INVENTION

The present invention relates to generation of specific antigens associated with P. multocida under controlled culture conditions and the use of such conditions in monitoring the generation of specific antigens.

The invention relates to antigens preferably derived from culture supernatant, and most preferably the invention relates to identifying and purifying antigens from culture supernatant of a culture of P. multocida.

The present invention also relates to these antigens and the use of such antigens in the detection and purification of a number of protective and diagnostic antigens and antibodies from parasites and particularly bacteria. In particular, the present invention relates to the identification and purification of antigens in the bacterium family Pasteurellae, preferably Pasteurella multocida, and most preferably Pasteurella multocida type A:3.

The invention also relates to the use of the antigens in vaccines and in assays for diagnostic purposes.

The vaccines of the invention have antigens produced in accordance with the invention, preferably the vaccines have antigens derived from a culture supernatant of a culture of bacterium growth under controlled conditions, and more preferably, growth under iron-restricted culture conditions.

### BRIEF DESCRIPTION OF THE DRAWINGS

IN THE FIGURES:
Figure 1 : Growth of P. multocida. Growth curves of P. multocida illustrating (a) growth measured with respect to optical density over time. Optical density was measured at 525nm. (b) Growth measured with respect to CFU/ml over time. Logarithmic growth was obtained between 120 mins & 220 mins.
Figure 2 : Screening of P. multocida culture supernatant and cells with immunized cattle sera and/or antigen screening supernatants. Supernatant from cultures of designated lymph nodes of Animals 19 and 25.
Figure 3 : Screening of P. multocida culture supernatant and cells with immunized cattle sera and antigen screening supernatants. Supernatant from cultures of designated lymph nodes of Animals 19, 15 and 20.
Figure 4 : Screening of P. multocida culture supernatant and cells with immunized cattle sera and antigen screening supernatants. Supernatant from designated lymph nodes by CAB technology of Animals 4 and 13.

### DETAILED DESCRIPTION OF THE INVENTION

In a first aspect, the present invention provides a method for producing at least one antigen of P. multocida species or related species, which method includes
providing a biological sample infected with P. multocida;
culturing the biological sample in a culture medium under suitable conditions;
harvesting the antigens from the culture at a suitable stage; and
optionally purifying and identifying an antigen associated with P. multocida.

The P. multocida may be from any one of the serogroups or somatic serotypes. Preferably the serogroup is any one of Type A, B, C, D, E or F, more preferably type A or D and most preferably type A. The somatic serotype may be any serotype, however, for Pneumonic pasteurolosis, it is most preferably Type A³.

The antigens produced according to the method described above may be utilized to identify and produce antibodies and antibody probes for the further detection of antigens in animals infected with a Pasteurellae or preferably P. multocida.

The biological sample infected with P. multocida may be a bacterial culture of P. multocida of any level of purity. Preferably it is a pure culture of P. multocida. Hence in a further aspect of the invention there is provided a method for producing at least one antigen of P. multocida species or related species which method includes
providing a colony of P. multocida;
isolating cells of P. multocida from the colony;
culturing the cells from the colony in a culture medium under suitable conditions;
harvesting antigens from the culture at a suitable stage of growth; and
optionally purifying and identifying an antigen associated with P. multocida.

The culturing of cells may include several cultural passages under conditions suitable to induce growth of P. multocida.

Conditions for the growth of the cells may be standard conditions for the growth of the biological sample or bacterium involved.

For the culture of P. multocida the colony may be provided on any suitable growth medium, preferably blood agar plates.

Isolation of cells from a colony may be achieved by any known selection methods available to the skilled addressee.

The culturing of the P. multocida bacteria may be carried out in any culture medium suitable to induce growth of the cells. Preferably, the growth medium is a minimal bacterial growth medium and more preferably it is BHIB (Brain Heart Infusion Broth) or a BHIB containing medium. Most preferably, it is a combination of media, preferably a mixture of BHIB and RPMI 1640.

The culture is preferably supplemented with a nutrient source, such as sucrose, glucose, or serum. Most preferably, the culture is supplemented with sucrose.

The conditions for growth of the cells from colonies will be sufficient and suitable for the P. multocida to grow and express antigens. As discussed above, P. multocida has the ability to acquire iron during the disease process. The level of iron may have an effect on the reaction of P. multocida to the host defense mechanisms. Thus, the growth medium, preferably, has a low concentration of iron to stimulate the P. multocida iron scavenging system.

Accordingly, in another aspect of the present invention there is provided a method for producing at least one antigen of P. multocida species or related species which method includes
providing a biological sample infected with P. multocida;
culturing the biological sample in an iron-regulated culture condition in a suitable culture medium;
harvesting the antigens from the culture at a suitable stage; and
optionally purifying and identifying an antigen associated with P. multocida.

The iron-regulated culture condition may be provided by a medium having a high or low concentration of Iron, however, the concentration of the iron will not be so high or so low as to prevent growth and expression of antigen. The iron regulation may be applied at any time during culturing of the biological sample. Iron regulation may be applied during all stages of culturing or at the stage of culturing prior to harvesting.

Accordingly, in a further aspect of the present invention, there is provided a method of producing at least one antigen of P. multocida species or related species which method includes
providing a biological sample infected with P. multocida;
culturing the biological sample under iron-restricted culture conditions in a suitable culture medium;
harvesting the antigen from the culture at a suitable stage; and
optionally identifying and purifying an antigen associated with P. multocida.

Restriction of the iron concentration may be controlled by the addition of low concentrations of iron or by the addition of a chelator. Preferably the chelator will be an iron chelator such as Transferin, ferritin, ferrodoxin, EDTA, EDDA or Dipyridyl.

Preferably, the Iron chelator is 2,2'Dipyridyl.

The antigen may be harvested at any time during the culturing period. The culture may be monitored to determine a suitable stage at which the antigen is to be harvested. The stage may be determined by any method which identifies an optimal time of harvest. The optimum time to harvest cultured supernate in isolating the antigen for a vaccine is preferably during active log phase and more preferably early to mid logarithmic growth of the P. multocida when sufficient growth has occurred to generate sufficient proteins and antigens. At least one log of growth is required to obtain such levels.

It has been found that growth in the medium is sufficiently variable that a characteristic of a determinate of the growth of the bacterium must be monitored rather than specifying the specific time during growth of the bacterium at which harvest is to take place. A useful determinant, according to this preferred embodiment, is optical density or a measurement of the number of colony forming units/ml (CFU/ml). Preferably, the optical density is measured at a wavelength of 525 nm. In a fresh culture of P. multocida which has been innoculated the optical density measured a 525 nm may be in the range of 0.01 to 0.03, preferably 0.02 to 0.03. The cells must undergo at least one log of growth, preferably two logs of growth to generate sufficient protein and antigen however, if excessively more, then the growth will exceed active log phase and harvest will not occur.

Once this stage is reached, and harvest occurs, then preferably the optical density is in the range of about 0.2 to 0.4, more preferably about 0.25 to 0.35 and most preferably in the range of about 0.28 to 0.32.

Harvesting the antigen may be performed by centrifugation of the culture to separately isolate the supernatant and the cells or bacteria from which the antigens may be harvested. Harvesting of the antigens in culture includes harvesting antigens in the culture supernatant as well as the cells. After harvesting the supernate liquid containing the antigen from the medium, a suitable vaccine may be prepared from the harvested liquid. Moreover, harvesting of the supernatant during active log phase closely mimics the in vivo growth of the organism.

At harvest, the liquid is processed to remove extraneous matter. For example, the harvested liquid may be centrifuged further and filtered to remove all solids which include cells, cell wall fragments, unwanted metabolites and the like, thereby providing a liquid which is cell free and which is relatively endotoxin free. Thus, when the vaccine is administered, the likelihood of anaphylactoid reactions is minimized which is a problem with prior vaccines of this nature due to the presence of endotoxin in the cell wall of the gram negative bacterium P. multocida. Since this organism has a thick cell wall and capsule, antigens trapped beneath the capsules are released into the supernatant without the cell wall to be present in a vaccine.

It is appreciated that variety of suitable techniques are available for isolating the antigens and preparing the vaccine which are readily known to those skilled in the art. The determination of suitable techniques is dependent upon the characteristic of the product to be prepared and the scale of commercialization.

The supernatant or cells may be treated in any way to concentrate antigen. This includes filtration, evaporation, centrifugation or lyophilization.

The antigens so harvested in culture supernatant or cells may be used at this stage with or without concentration as a purified liquid to prepare vaccines. Such a vaccine may be used to raise antibodies including monoclonal or polyclonal antibodies, or used to construct recombinant protein antigens for use in place of native antigens or simply the antigen may be separated, purified, isolated and characterized. The antigens may be used for diagnostic assays and kits to detect infection by P. multocida or used as a parameter to monitor culture conditions effective to induce production of such antigens.

The antigens may be separated and purified by any known conventional means which include the use of electrophoretic or chromotography techniques including gel chromatography, affinity chromatography, and SDS acrylamide gels.

The antigen contained in the culture medium may be detected using any suitable assay technique which may include Western Blot techniques, immunoprecipitation assays, radioimmunoassay or enzyme-linked immuno assays (ELISA) or immunofluorescent assays. The antigen may be detected using antibody probes derived from animals infected with the bacterium. The animals may be infected by innoculation of the bacterium or may be from natural infection.

Antibody probes may be derived from infected serum obtained from an animal innoculated or naturally infected with the bacterium, or the antibody probes may derive from supernatant of a culture of a biological sample of an infected animal.

The biological sample may be of any suitable type. The biological sample may be from an animal infected with P. multocida. The biological sample may be from infected animal tissues, organs, blood, including serum or plasma, lymph or lymph nodes. The biological sample may be taken from any section of the infected animal. However, it is preferred that the samples be taken from the infected site or an area of a lesion which may be formed in certain diseases or an area close to the infected site or a lesion such as in the lymph nodes. Preferably samples may be taken from the hepatic lymph node, abomasal lymph node, mesenteric lymph node or lymph nodes in the lung.

The biological sample may be taken any time from the animals, preferably they are taken a short time after the animal has been infected or when the animal has developed symptoms of the invention.

More preferably the antibody probes are produced by a method described in Australian patent 640364, the entire disclosure of which is incorporated herein by reference. This patent describes the development of an antiobdy probe effective for identifying antigens associated with a disease pathogen. The method is appliable for developing antibody probes to P. multocida antigens. The technique is known herein as CAB technology. (Centre for Animal Biotechnology Technology).

Accordingly, in a preferred aspect, the present invention provides a method for producing at least one antigen of P. multocida species or related species, which method includes:
providing a colony of P. multocida;
isolating cells of P. multocida from the colony;
culturing the cells from the colony in a culture medium under suitable conditions;
harvesting antigens from the culture at a suitable stage of growth; and
purifying and identifying an antigen associated with P. multocida using a P. multocida antibody probe or functional equivalent.

In a further preferred aspect, the present invention provides a method for producing at least one antigen of P. multocida species, or related species, which method includes:
providing a colony of P. multocida;
isolating P. multocida cells from the colony;
culturing the cells from the colony in an iron-regulated culture condition in a suitable culture medium;
harvesting the antigens from the culture at a suitable stage; and
purifying and identifying an antigen associated with P. multocida using P. multocida antibody probe or functional equivalent from an animal infected with P. multocida.

In a more further preferred aspect of the present invention there is provided a method for producing at least one antigen of P. multocida species or related species which method includes
providing a colony of P. multocida;
isolating P. multocida cells from the colony;
culturing the cells from the colony under iron-restricted culture conditions in a suitable culture medium;
harvesting the antigen from the culture at a suitable stage; and
identifying and purifying an antigen associated with P. multocida using P. multocida antibody probe or functional equivalent developed by CAB technology.

Accordingly, a further aspect of the invention provides antigenic proteins associated with P. multocida having molecular weights in the range of about 60K, 40-42K, 35K and 14K.

The antigens so obtained may be coupled to a suitable solid support eg. CNBr-activated sepharose 4B (Pharmacia), Affigel (BioRad) or other affinity chromatography supports able to bind proteins.

Accordingly, in a preferred aspect of the present invention there is provided a further method of producing an antibody to an antigen associated with P. multocida, which method includes
providing an antigen associated with P. multocida prepared by a process as discussed above;
immobilizing said antigen on a solid support;
subjecting said immobilizing antigen to a sample containing antibodies to P. multocida and allowing the antibody and antigen to react and bind;
removing unreacted sample; and
eluting bound antibody from said antigen.

Immobilized antigen can be applied to fractionation and purification of specific antibodies from a complex serum sample or culture supernatants of immune cells from infected animals by affinity chromatography. The serum sample may be obtained from serum of infected animals infected with the bacterium P. multocida.

After binding of antibody to immobilised antigen, unbound macromolecular species can be washed away from the solid support with, e.g. buffers containing 1.5M NaCl. Subsequently, the antigen can be eluted from the affinity column with, e.g. low or high pH buffer or buffers containing chaotropic ions, e.g. 0.5 - 3.0 M sodium thiocyanate.

Accordingly, there is provided in the present invention an antiobdy to an antigen of P. multocida prepared by the method described above.

The antigens isolated or located may be used in the preparation of monoclonal antibodies. The monoclonal antibodies may form the basis of a passive treatment of the disease discussed above.

In another aspect, there is provided a monoclonal or polyclonal antibody against a protective antigen as hereinbefore described, or fragment thereof, against P. multocida species, and related species.

The antibody may be produced by a method which includes providing
a B cell capable of producing antibodies against said protective antigen, or fragments thereof, and obtained from an animal immunized with a protective antigen from P. multocida as described above; and
a myeloma cell;
fusing the B cell with the myeloma cell;
propaging a hybridoma formed thereby, and
harvesting the antibody produced by said hybridoma.

Having identified the antigen(s), molecular biology or chemical techniques, e.g. cloning techniques may be used to produce unlimited amounts of this antigen or alternatively synthetic peptides corresponding to different fragments of the identified antigens may be used as a means to produce a vaccine.

Accordingly, in a preferred aspect of the present invention there is provided a method for preparing a synthetic antigenic polypeptide associated with P. multocida species, and related species, which method includes providing
a genomic library derived from a biological sample of P. multocida species, and related species; and
a corresponding antibody selected from the group consisting of
a antibody as described above; or
a monoclonal antibody, or a derivative thereof as described above; or
an antibody probe as described above; generating synthetic polypeptides from the cDNA library or genomic library;
probing the synthetic polypeptides with the antibody probe; and
isolating the synthetic antigenic polypeptide detected thereby.

Genomic libraries may be used. The genomic libraries may be assembled into suitable expression vectors that will enable transcription and the subsequent expression of the clone of DNA, either in prokaryotic hosts (e.g. bacterial) or eukaryotic hosts (e.g. mammalian cells). The probes for screening the libraries may preferably be selected from:
(i) synthetic oligonucleotide probes based on segments of the full amino acid sequence of the antigen identified and purified;
(ii) antibodies based on synthetic peptides corresponding to amino acid sequence data of the antigen identified;
(iii) antibodies obtained from the culture medium;
(iv) monoclonal or polyclonal antibodies produced against the antigens identified and purified; and
(v) recombinant or synthetic monoclonal antibodies or polypeptides with specificity for the antigen, e.g. as described by Ward et al. 1989, Nature 241, pages 544 to 546.

The antigen isolated may be a protective antigen effective as a vaccine against subsequent infection by P. multocida or used in diagnostic assays as described below.

In a further aspect the present invention provides a nucleic acid sequence encoding an approximately 60K, 40-42K, 35K or 14K protective antigen from P. multocida or related microorganism; or a functionally active analogue, mutant, derivative or fragment thereof.

Accordingly, in a further aspect of the present invention, there is provided a protective antigen against P. multocida species or related species prepared by a method including a method of producing at least one antigen of P. multocida species or related species which method includes
providing a colony of P. multocida;
isolating P. multocida cells from the colony;
culturing the cells from the colony under iron-restricted culture conditions in a suitable culture medium;
harvesting the protective antigen from the culture at a suitable stage; and
optionally identifying and purifying a protective antigen associated with P. multocida.

Accordingly, in a further preferred aspect of the present invention there is provided a protective antigen, or antibody-inducing fragment thereof, against P. multocida infections prepared by any of the methods outlined above. Preferably the antigen is selected from antigens having approximate molecular weights in the range of about 60K, about 40-42K, about 35K and about 14K. The antigens may also be present in other species and strains of the bacterium.

It will be also understood that recombinant protein antigens may be used in place of the native antigens extracted from the bacterium as protective antigens.

Moreover, such antigens may be combined with other antigens, or fragments thereof, into a polyvalent vaccine, e.g. with antigens isolated from cultures of P.haemolytica, to treat Shipping Fever. Such antigens may be obtained according to the methods described in United States Patent Nos. 5,055,400 and 5,165,924.

Fragments of the antigen(s) containing protective epitopes, and synthetic peptides containing protective epitopes may be substituted for the entire native or recombinant molecule(s) in both vaccines and diagnostic tests and kits incorporating those tests.

The antigen(s) may be present in other species of P. multocida and would thus be of use in vaccination and diagnosis of other diseases in addition to that caused by the designated pathogens.

In another aspect the present invention provides a method for preventing infection caused by P. multocida species, and related species, in animals, which method includes administering to an animal an effective amount of at least one protective antigen prepared by a method as described above.

Preferably the protective antigen is an antigen derived from P. multocida type A and more preferably from P. multocida type A³.

In another aspect, the present invention provides a method for the treatment of infection caused by a P. multocida species, and related species, which method includes administering to an animal an effective amount of at least one protective antigen as described above.

It will be further understood that antibody raised against these antigens may be used in diagnostic tests or as an immunoprophylactic agent, whether polyclonal or monoclonal.

In a still further aspect of the present invention there is provided a method for the treatment of infection caused by a P. multocida species, and related species, in animals, which method includes administering to an animal a therapeutically effective amount of a monoclonal or polyclonal antibody to a protective antigen produced as described above.

The present invention further provides a vaccine composition including a prophylactically effective amount of at least one antigen from P. multocida species, and related species in a pharmaceutically acceptable carrier.

The present invention further provides a vaccine or veterinary composition including a therapeutically effective amount of at least one monoclonal or polyclonal antibody against a protective antigen as described above in a veterinarily acceptable carrier.

The vaccine or veterinary compositions according to the present invention may be administered orally or may be administered parenterally (for example by intramuscular, subcutaneous, intradermal or intravenous injection).

The amount required will vary with the antigenicity of the active ingredient and need only be an amount sufficient to induce an immune response typical of existing vaccines.

Reactive experimentation will easily establish the required amount. Typical initial doses of vaccine or veterinary compositions may be approximately 0.001-1 mg active ingredient/kg body weight. The dose rate may increase or multiple doses may be used as needed to provide the desired level of protection.

The vaccine or veterinary composition according to the present invention may further include a veterinary acceptable carrier, diluent or excipient thereof. Preferably the active ingredient may be suspended or dissolved in a carrier. The carrier may be any solid or solvent that is non-toxic to the animal and compatible with the active ingredient. Suitable carriers include liquid carriers, such as normal saline and other non-toxic salts at or near physiological concentrations, and solid carriers, such as talc or sucrose. Adjuvants, such as Freund's adjuvant, complete or incomplete, munokynin (Quil A), NVA (Carbopol) or immunomodulators, such as cytokines, may be added to enhance the antigenicity of the antigen if desired. When used for administering via the bronchial tubes, the vaccine is suitably presented in the form of an aerosol.

In a still further aspect of the present invention there is provided a diagnostic assay kit including a diagnostic antigen, or fragment thereof, against P. multocida identified and purified as described above.

The diagnostic kit may be utilised to detect infections in animals caused by P. multocida species or related species.

The diagnostic assay kit may be utilised in conjunction with a diagnostic assay. The diagnostic assay may include western blot techniques. The diagnostic assay may be a diagnostic immunoassay. The immunoassay may be an immunoprecipitation assay, a radioimmunoassay, an enzyme linked immunoassay, an immunofluorescent assay or a chemiluminescent assay.

The technology described herein may also be used as a means of monitoring culture conditions in a culture of P. multocida. The expression of antigens are a reflection of the culture conditions presented to the organism. By monitoring the level of an antigen production, one can deduce the culture conditions presented to the organism which results in the production or production rate of a specific antigen.

Accordingly, there is provided a method of monitoring culture conditions in a culture of P. multocida including:
obtaining a sample of the culture of P. multocida; and
monitoring an antigen of P. multocida.

The antigen may be any antigen of P. multocida. Preferably it is an antigen that is responsive under certain culture conditions, more preferably it is an antigen which is generated by the bacterium under iron-regulated conditions and more preferably, under iron-restricted conditions. Preferably, the antigen is any one of the P. multocida antigens produced under iron-restricted conditions including antigens of about 60KD, 40-42K, 35K or 14K molecular weight.

Monitoring of the antigen may be by any detection means including the use of antibodies, monoclonal or polyclonal antibodies, or antibody probes which are labelled or conjugated with a detector molecule.

Conversely, by measuring the culture conditions, one can predict the type of antigen produced. Hence, culture conditions may reflect the antigen production.

Accordingly, there is provided a method of monitoring antigen production including:
obtaining a sample of the culture of P. multocida; and
monitoring the culture conditions.

Also, antigens may be produced at different rates depending on culture conditions. Hence, measurment of at least one antigen in comparison to another antigen may also reflect the culture conditions. Conversely, measurement of the culture condition can provide an insight to the level of production for an antigen in comparison with another antigen.

Accordingly, there is provided a method of monitoring culture conditions including:
measuring the level of at least one antigen of P. multocida and comparing the level with the level of at least one other antigen of P. multocida under similar culture conditions.

There is also provided a method of monitoring antigen production including measuring the production of at least one antigen and comparing the production of that antigen to deduce the level of another antigen produced under similar conditions.

The present invention will now be more fully described with reference to the following examples. It should be understood, however, that the description following is illustrative only and should not be taken in any way as a restriction on the generality of the invention described above.

### EXAMPLE 1

### 1.1 Pasteurella multocida culture and antigen production

### 1.1.1 Cultivation

On Day 1, several colonies from a blood agar plate of P. multocida grown overnight at 37°C are innoculated into 250mls of brain-heart infusion broth (BHIB) and incubated at 37°C at 100 RPM for 6 hours.

After the 6 hour period, a new culture in a 1L flask is made for cultivation of P. multocida in Iron chelator medium. In a 1L flask, there is added, 250mls of 10% sucrose stock solution, 2.5ul 300mM stock solution of 2,2' Dipyridyl (prepared in 70% Ethanol), 10mls of BHIB culture, and 465mls of RPMI-1640. The flask is incubated at 37°C and 100 RPM overnight.

On Day 2 of the culture, the 1L culture is upscaled to a 2L fermenter or a 4L flask. To this was added 2-3% of the culture described above (1L culture in iron restricted conditions), 100mls of 10% sucrose stock solution, 10ul of 300mM stock solution of 2,2' Dipyridyl and 2000mls of RPMI-1640.

This culture is then incubated overnight at 37°C at 100 RPM in a 4L flask or at 37°C with a stirrer speed of 300 RPM in a fermenter.

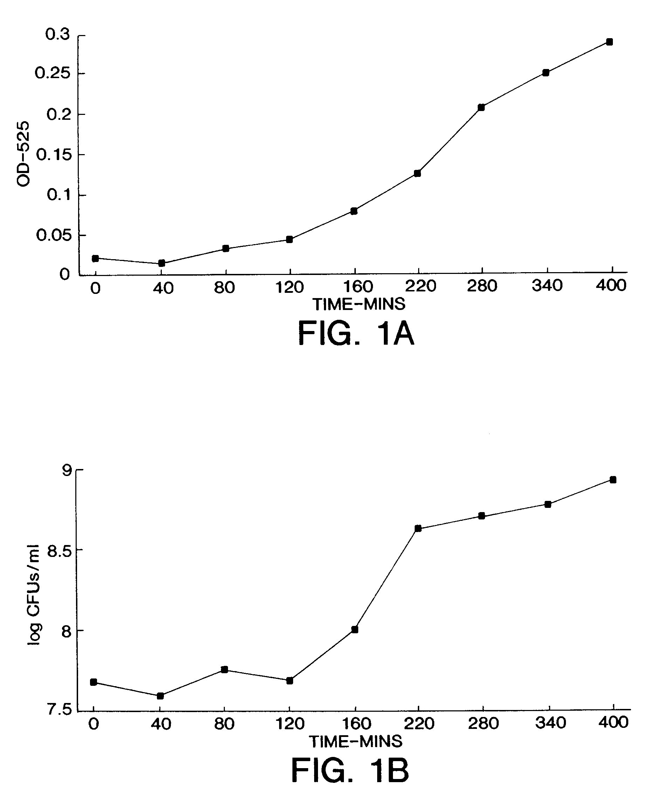

The start OD₅₂₅ is in the range of 0.01 to 0.03. Optical density is monitored at 525mM and the culture is harvested when the OD₅₂₅ is in the range of 0.28 to 0.32 after 3 to 3.5 hours when the organisms have gone through at least oen log of growth, as shown in Figure 1.

The culture is then transferred to centrifuge tubes (500mls) and centrifuged at 9500 RPM for 15 minutes at 4°C. The supernatant obtained from the centrifuge tubes is filtered through a 0.22um filter. The cells are collected at this stage as a cell pellet to prepare a cell lysate.

Dialysis tubing is soaked in ddH₂O for 5 to 10 minutes prior to use. Supernatant is then dialysed against 20 litres of ddH₂O at 4°C over two days with a change of ddH₂O in between.

After this period, supernatant is collected and the total volume measured before lypophilizing.

The lypophilized supernatant may be resuspended in sterile water to any desired concentration. A 1000mls of lypophilized supernatant may be resuspended in 10mls to give a 100X concentrated preparation.

This preparation is then used as a vaccine by blending with various adjuvants. This preparation represents antigens produced in response to cultivation in the presence of Iron restricted conditions, wherein the iron restriction is imposed by the addition of an Iron chelator such as Dipyridyl. Supernatants from cultures, without iron restriction, that is RPMI and sucrose only is also collected.

A cell suspension and lysate may also be prepared by known techniques to provide a source of antigens for use on a gel or in a vaccine.

### EXAMPLE 2

### PREPARATION OF ANTIBODY PROBES

### 2.1 Challenging/Vaccination of Calves to obtain immunity

Calves are innoculated with varying doses of P.multocida starting from Day O, as outlined below in Table 2-1 using P. multocida at 10⁵(Low Dose), 2.5 x 10⁷ (Medium Dose) and/or 10⁸⁻¹⁰ (High Dose) and/or P.multocida culture supernatant (vaccination) obtained as outlined in Example 1. After a period of Days set out in Table 2-1, the animals are further challenged with a high or medium dose of P. multocida. After 3-4 days following challenge the animals are sacrificed and the lung and lymph nodes removed and processed by CAB technology as outlined in 2.2 to produce an antigen screening supernatant. Serum from infected animals is also collected for screening culture supernatant as in Example 3.

**TABLE 2.1**

| **P.MULTOCIDA EXPERIMENTAL PROCEDURES** | | | |
|---|---|---|---|
| ANIMAL | DEVELOPMENT OF IMMUNITY | CHALLENGE FOR VACCINE TECHNOLOGY | TIME KILLED |
| 2 | LOW DOSE, DAY 0,7,13, 21, 28. | DAY 93 HIGH DOSE | DAY 96 (3 DAY) |
| 4 | VACCINATION DAY 0,21,29. LOW DOSE, DAY 15. HIGH DOSE, DAY 55. | DAY 103 HIGH DOSE | DAY 107 (4 DAY) |
| 13 | LOW DOSE, DAY 0, 7, 13, 21. | DAY 95 HIGH DOSE | DAY 99 (3 DAY) |
| 15 | LOW DOSE, DAY 0,7,13,21. | DAY 95 HIGH DOSE | DAY 98 (4 DAY) |
| 16 | LOW DOSE, DAY 0,7,13,21. | DAY 102 MEDIUM DOSE | DAY 105 (3 DAY) |
| 19 | VACCINATION, DAY 0,15,29. HIGH DOSE DAY 55. | DAY 104 HIGH DOSE | DAY 107 (3 DAY) |
| 20 | VACCINATION, DAY 0,15,29. HIGH DOSE DAY 55 | DAY 103 HIGH DOSE | DAY 106 (3 DAY) |
| 21 | VACCINATION, DAY 0,15,29. | DAY 110 MEDIUM DOSE | DAY 113 (3 DAY) |
| 24 | VACCINATION, DAY 0,15,29. | NONE (CONTROL) | DAY 103 |
| 25 | VACCINATION, DAY 0,15,29. | DAY 102 HIGH DOSE | DAY 105 (3 DAY) |

All animals receive Tilmicosin (micotil, 6 ml subcutaneously) on about days 40 and 66.

### 2.2 Processing bovine lymph nodes (CAB technology) to produce an Antigen Screening Supernatant

### (a) Reagents

1. DMEM - Liquid media in 500ml bottles
   Media contains low glucose 100mg/L
   D-Glucose, L-glutamine, 25mM Hepes, 100mg/L sodium pyruvate.
   Gibco Cat # 12320-024
   OR
   Powder DMEM - 10L prepared from one bottle
   Add to media 6g/L Hepes and 3.7g/L NaHCO₃
   Filter sterilize
   Gibco Cat # 31600-083
2. L-Glutamine 200mM - ICN/Flow Cat # 16-801-49
3. 2 x Pen/Strep 10000 IU/ml & 10000 MCG/ml - ICN/Flow Cat # 16-702-49
4. Foetal Calf Serum (FCS) - Gibco BRL - Cat # 16000-028 - Heat inactivate (HI) at 56°C for 1Hr.
5. 2-Mercaptoethanol (2-ME) - Prepare stock solution of 160ul of 2-ME in 10mls distilled water. Filter sterilize.
6. PBS + 5mM EDTA
7. PBS
8. 70% Ethanol
9. Trypan Blue stain - 0.05% in PBS + 0.05% Azide

| Media recipes | |
|---|---|
| Growth media | Wash media |
| DMEM - 500mls | DMEM - 500mls |
| 10% FCS (HI) - 50mls | Glutamine - 5mls |
| Glutamine - 5mls | Pen/Strep - 10mls |
| Pen/Strep - 10mls | 2-ME - 100ul of stock |
| 2-ME - 100ul of stock | |

### (b) Processing the lungs

Lung is removed under sterile conditions and transported on ice. The lungs are washed with PBS and placed on a sterile tray for dissection. Bronchial and mediastinal lymph nodes are identified, location noted and dissected.

The nodes are dissected and stored in a container with wash media and placed on ice. About 5 to 6 lymph nodes are usually obtained. At this stage, a piece of lesioned lung is also kept and stored in a container with PBS-EDTA and placed on ice.

### (c) Processing the lymph nodes

Lymph nodes are aseptically removed from the container and placed in a petri dish lid. Fat layers attached to the lymph nodes are removed with sterile forceps and scissors. The lymph nodes are then dissected and teased through a strainer to release cells. The cells are washed and centrifuged at 1200 RPM for 10 minutes at 4°C. Supernatant is removed and the cells resuspended in growth media.

### (d) Processing the lung tissue

Lung tissue is washed and flushed with PBS-EDTA. The lung is dissected and teased through a seive with PBS. The cells are washed at 1200 RPM for 10 minutes at 4°C and the supernatant removed before resuspending cells in PBS-EDTA. The cells are repeatedly washed 3 to 4 times by resuspending and centrifuging at 1200 RPM for 10 minutes at 4°C. Cells are then resuspended in 40mls of growth media and stored on ice.

### (e) Setting up tissue culture flasks

Cells are counted on a haemocytometer and resuspended in growth medium at 5 x 10⁸ cells/ml. Tissue culture flasks are seeded at 5 x 10⁶ cells/ml in growth media to a total volume of 50mls in a 75cm² flask or 10mls in a 25cm² flask. Cells are incubated at 37°C with 5% CO₂. After 3 or 4 days incubation, cells are collected at 3000 RPM for 15 minutes. Supernatant was collected and stored at -20°C. This is the antigen screening supernatant or CAB vaccine.

### EXAMPLE 3

### 3.1 Identification of antigens in P. multocida culture supernatant and cells

Culture supernatant and cell lysate as prepared in Example 1 are run on SDS-PAGE with 12% separating gel and 4% stacking gel.

Protein is transferred onto nitrocellulose membranes after soaking the membrane in methanol. The membrane is then incubated in block buffer containing 500mP PBS, 500ul FCS and 250mP Tween-20 for one hour.

The membrane is incubated in 2mls of Antigen Screening Supernatant or CAB vaccine [Example 2.2] or 1/20 dilution of sera of an infected animal for 3 hours and then washed 3 times in block buffer. Membrane is then incubated with a 1/2000 dilution of a goat anti-bovine alkaline phosphatase conjugated antibody for one hour followed by 2 washes in block buffer and one wash in carbonate buffer. Substrate is added to develop colour for detection of bands.

**TABLE 3.1**

| **RESULTS OF CAB VACCINE TECHNOLOGY APPLIED TO P.MULTOCIDA** | | | |
|---|---|---|---|
| ANIMAL | DEVELOPMENT OF IMMUNITY | ANTIGENS IN CULT. SUPT. | ANTIGENS IN CELLS |
| 2 | LOW DOSE | MANY ANTIGENS - NONSPECIFIC | MANY ANTIGENS - NONSPECIFIC |
| 4 | VACCINATION | 80kD,55-60kD, | -62kD,38-40kD, |
| | LOW DOSE | 38-40kD | - 14kD |
| | HIGH DOSE | (2-SUPTS EACH) | |
| 13 | LOW DOSE | MANY BANDS 110,80st,58,88 | MANY BANDS 58-60st,40,37-8 9st |
| 15 | LOW DOSE | 42kD (1 SUPT) | 42kD(3 SUPTS) 58kD(1 SUPT) |
| 16 | LOW DOSE | | |
| 19 | VACCINATION | - 14kD SMEAR | (86),66(1 SUPT) |
| | HIGH DOSE | 42kD v. weak | 42kD(2 SUPT) |
| 20 | VACCINATION | 42kDst(ALL SUPT) | 42kDst(ALL SUPTS) |
| | HIGH DOSE | 14kD SMEAR OTHER W. BANDS | OTHER WEAK BANDS |
| 21 | VACCINATION | | |
| 24 | VACCINATION (UNCHALL. CONTROL) | NONE | NONE |
| 25 | VACCINATION | GEN. -ve 18kD(1 SUPT) | 65w,44w,40st, 33st,28w,18kD-s t.OTHER WEAK BANDS |
| st = STRONG BAND ON WESTERN. w = WEAK BAND ON WESTERN. | | | |

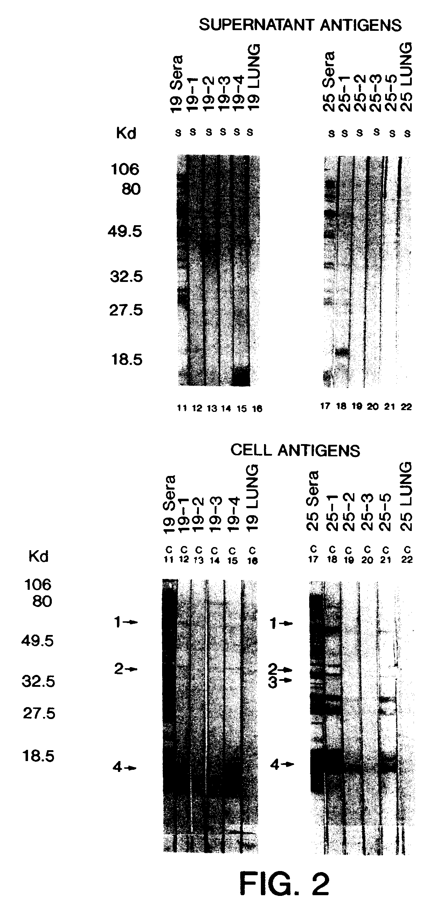

Figure 2 shows Western blots of P. multocida supernatant (Example 1) and P. multocida cells (Example 1) grown in the presence of dipyridyl and screened with antigen screening supernatant obtained by CAB technology or sera from calves No. 19 and No. 25. The first (left hand) strip for each set is probed with serum and the remaining strips with antigen screening supernatants from individual lymph nodes or from lung tissue (LLO).

### Identified antigens are labelled

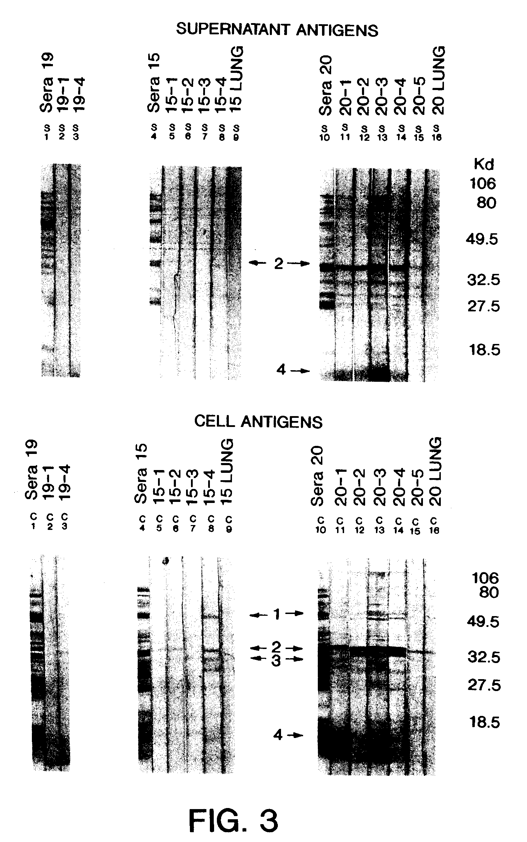

1 = 60 kDA
2 = 40-42 kDA
3 = 35 kDA
4 - 14 kDA
Figure 3 shows Western blots of P. multocida supernatant (Example 1) and P. multocida cells (Example 1) grown in the presence of dipyridyl and screened with antigen screening supernatant obtained by CAB technology or sera from calves No. 15, No. 19 and No. 20. The first (left hand) strip for each set is probed with serum and the remaining strips with antigen screening supernatants from individual lymph nodes or from lung tissue (LLO).

### Identified antigens are labelled

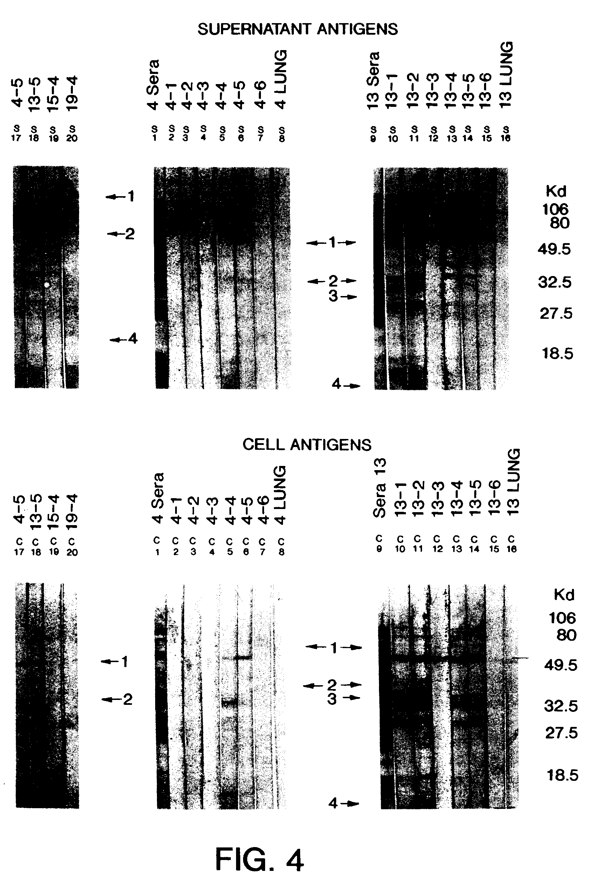

1 = 60 kDA
2 = 40-42 kDA
3 = 35 kDA
4 - 14 kDA
Figure 4 shows Western blots of P. multocida supernatant (Example 1) and P. multocida cells (Example 1) grown in the presence of dipyridyl and screened with antigen screening supernatant obtained by CAB technology or sera from calves No. 4 and No. 13. The first (left hand) strip for each set is probed with serum and the remaining strips with antigen screening supernatants from individual lymph nodes or from lung tissue (LLO).

### Identified antigens are labelled

1 = 60 kDA
2 = 40-42 kDA
3 = 35 kDA
4 - 14 kDA

### 3.2 Results of CAB vaccine technology

1. 40-42kD antigen is the most consistently and strongly identified antigen from both supernatant and whole cell antigens.
2. Several other antigens have also been identified. In particular a - 60kD antigen in cells and supernatant, and a low MW smear of - 14kD that is strongly recognised by a number of animals in supernatant. The 14kD antigen does not resolve well on SDS-PAGE and may be charged polysaccharide or LPS. Other antigens (35k) have been less consistently identified with the limited number of clearly responding animals.

### 3.3 Summary

The following 3 antigens are identified:
(i) a 42kD antigen found in cells and culture supernatant.
(ii) a - 60kD antigen found in cells and culture supernatant.
(iii) a - 14kD antigen predominantly found in culture supernatant.
A minor antigen at 35kD is also recognised by several cattle.

### EXAMPLE 4

### 4.1 Effect of supernatant as a vaccine

A vaccine prepared from the culture supernatant may be prepared according to Example 1 and animals were vaccinated and challenged with the microorganism.

**Table 4.1**

| Effect of P. multocida vaccine on Body Weight between challenge and necropsy | | | |
|---|---|---|---|
| | Control | P.multocida | P.multocida + C |
| n | 8 | 9 | 9 |
| Change in BW | -0.25 | 9.9 | 24.1 |
| Change in BW = Change in weight over the post-challenge period (weight day 47 - weight day 41) Control: PBS - 2 doses, 2ml/dose IM, at day 0 and 21 (3 week interval). P.multocida: Pasteurella multocida experimental vaccine, 2 doses, 2ml/dose IM, at day 0 and 21 (3 week interval). P.multocida + Chelator (C): Pasteurella multocida + chelator experimental vaccine, 2ml/dose IM, at day 0 and day 21 (3 week interval). 024-BI-CN-0193 | | | |

**Table 4.2**

| Effect of P. multocida vaccine on percent pneumonic tissue at necropsy | | | | |
|---|---|---|---|---|
| | Group 1 | Group 2 | Group 3 | Controls |
| n | 20 | 19 | 20 | 19 |
| Arcsin transformed | 30.5^{a} | 34.7^{a} | 30.1^{a} | 48.7^{b} |
| % pneumonic tissue (SE) | (17.3) | (22.5) | (20.1) | (19.9) |
| Least square means in the same row with different superscripts differ significantly (P < 0.006) Group 1 - P.multocida bacterial extract - 20 X concentration Group 2 - P.multocida bacterial extract - 12.5 X concentration Group 3 - P.multocida bacterial extract - 7.5 X concentration | | | | |

**Table 4.3**

| Effect of vaccine group on percent pneumonic tissue at necropsy | | | |
|---|---|---|---|
| | Control | P.multocida | P.multocida + C |
| n | 8 | 9 | 9 |
| Arcsine | 55.06^{a} | 33.83^{b} | 22.21^{b} |
| transformation of % pneumonic tissue (SE) | (4.68) | (4.41) | (4.41) |

| | | | |
|---|---|---|---|
| ^{a,b} Least-square means in the same row with different superscripts differ significantly (P < 0.02). | | | |
| Control: PBS - 2 doses, 2ml/dose IM, at day 0 and 21 (3 week interval). P.multocida: Pasteurella multocida experimental vaccine, 2 doses, 2ml/dose IM, at day 0 and 21 (3 week inveral) (absence of chelator). P.multocida + C: Pasteurella multocida + chelator experimental vaccine, 2ml/dose IM, at day 0 and day 21 (3 week interval). | | | |

**Table 4.4**

| Effect of vaccine group on clinical scores | | | | |
|---|---|---|---|---|
| | Group 1 | Group 2 | Group 3 | Controls |
| n | 20 | 19 | 20 | 19 |
| prechallenge clinical score | 0.2 | 0.23 | 0.18 | 0.19 |
| postchallenge clinical scores | 0.45^{a} | 0.67^{a} | 0.48^{a} | 1.35^{b} |
| Least square means in the same row with different superscripts differ significantly (P < 0.02) Group 1 - P.multocida bacterial extract - 20 X concentration Group 2 - P.multocida bacterial extract - 12.5 X concentration Group 3 - P.multocida bacterial extract - 7.5 X concentration | | | | |

**Table 4.5**

| Effect of vaccine group on percent pneumonic tissue at necropsy | | |
|---|---|---|
| | Vaccinates | Controls |
| n | 11 | 13 |
| arcsin transformed % | 31.7^{a} | 75.3^{b} |
| pneumonic tissue (SE) | (17.5) | (24.4) |
| Least square means in the same row with different superscripts differ significantly (P < 0.0001). Vaccinates = P. haemolytica toxoid/P.multocida bacterial extract - 20 X concentration | | |

Finally, it is to be understood that various other modifications and/or alterations such as preparation of an attenuated vaccine based on the description herein, may be made without departing from the spirit of the present invention as outlined herein.

## Claims

1. A method for producing at least one antigen of P. multocida species or related species, which method includes
providing a biological sample infected with P. multocida;
culturing the biological sample in a culture medium under suitable conditions;
harvesting the antigens from the culture at a suitable stage; and
optionally purifying and identifying an antigen associated with P. multocida.

2. A method for producing at least one antigen of P. multocida species or related species according to claim 1 wherein the biological sample includes cells of P. multocida isolated from a colony of P. multocida.

3. A method for producing at least one antigen of P. multocida species or related species according to claim 2 wherein the colony of cells is cultured in an iron-controlled culture condition in a suitable culture medium.

4. A method of producing at least one antigen of P. multocida species or related species according to claim 3 wherein the colony of cells is cultured under iron-restricted culture conditions in a suitable culture medium.

5. A method according to claim 4 wherein the culture medium includes an iron-chelator to provide an iron restricted culture condition.

6. A method according to claim 5 wherein the iron-chelator is dipyridyl.

7. A method according to claim 6 wherein the dipyridyl is 2,2'-dipyridyl.

8. A method according to any one of the preceding claims wherein a suitable stage to harvest the antigen is determined by measuring the optical density of the culture at 525 nm.

9. A method according to claim 8 wherein a suitable stage to harvest occurs at a stage when at least one log of growth has occurred since immunoculation of the culture medium with the colony of cells.

10. A method according to claim 9 wherein the optical density at 525 nm measured at harvest is in the range of 0.2 to 0.4.

11. A method according to claim 10 wherein the optical density is in the range of about 0.25 to 0.35.

12. A method according to claim 11 wherein the optical density is in the range of about 0.28 to 0.35.

13. A method according to any one of the preceding claims wherein the antigen is harvested from supernatant of the culture.

14. A method according to any one of claims 1 to 12 wherein the antigen is harvested from the cells of the culture.

15. A method according to any one of the preceding claims further including the step of identifying and purifying the antigens using an antibody probe derived from an infected animal.

16. A method according to claim 15 wherein the antibody probe is derived using CAB technology.

17. A method according to claim 15 wherein the antibody probe is derived from serum of an infected animal.

18. An antigen associated with P. multocida prepared by a method according to any one of the preceding claims.

19. An antigen associated with P. multocida according to claim 18 selected from the group consisting of those antigens having a molecular weight in the range of about 60K, 40-42K, 35K or 14K.

20. An antigen preparation including at least one antigen associated with P. multocida according to claim 18 or claim 19.

21. A method of producing an antibody to an antigen associated with P. multocida, which method includes
providing an antigen associated with P. multocida prepared by a method according to claim 4;
immobilising said antigen on a solid support;
subjecting said immobilised antigen to a sample containing antibodies to P. multocida and allowing the antibody and antigen to react and bind;
removing unreacted sample; and
eluting bound antibody from said antigen.

22. An antibody to P. multocida prepared by the method according to Claim 21.

23. A monoclonal or polyclonal antibody against an antigen of P. multocida species or related species or fragment thereof, produced by a method which method includes:
providing a B cell capable of producing antibodies against an antigen produced by a method according to claim 4, or fragments thereof, said B-cell obtained from an animal immunised with the antigen against P. multocida; and
a myeloma cell;
fusing the B cell with the myeloma cell;
propaging a hybridoma formed thereby, and
harvesting the antibody produced by said hybridoma.

24. A monoclonal antibody against P. multocida prepared by the method according to claim 23.

25. A method for preparing a synthetic antigenic polypeptide associated with P. multocida species, and related species, which method includes providing
a genomic library derived from a sample of P. multocida species, or related species; and
a corresponding antibody selected from the group consisting of
an antibody according to claim 22; or
a monoclonal antibody according to claim 24; or
an antibody probe prepared by CAB technology;
generating synthetic polypeptides from the genomic library;
probing the synthetic polypeptides with the antibody, monoclonal antibody or antibody probe; and
isolating the synthetic antigenic polypeptide detected thereby.

26. A protective antigen against P. multocida species or related species prepared by a method as claimed in any one of claims 1 to 17,

27. A protective antigen according to claim 26 selected from the group consisting of those antigens having a molecular weight in the range of about 60K, 40-42K, 35K or 14K.

28. A protective antigen preparation including at least one antigen associated with P. multocida according to claim 26.

29. The use of an effective amount of at least one protective antigen according to claim 26 to prevent infection caused by P. multocida species, and related species, in animals

30. The use of an effective amount of at least one protective antigen according to claim 26 to treat infection caused by P. multocida species, and related species, in animals.

31. The use of a therapeutically effective amount of a monoclonal or polyclonal antibody to a protective antigen according to claim 26 to treat infection caused by a P. multocida species, and related species, in animals.

32. A vaccine composition including an effective amount of at least one antigen from P. multocida species, and related species prepared by a method according to any one of claims 1 to 17 in a pharmaceutically acceptable carrier.

33. A vaccine or veterinary composition including a therapeutically effective amount of at least one monoclonal or polyclonal antibody against a protective antigen in a pharmaceutically or veterinarily acceptable carrier said monoclonal or polyclonal antibody prepared by the method according to claim 23.

34. A cell-free vaccine effective against pneumonic pasteurellosis in animals comprising;
a protective amount of a non-toxic inactive antigen specific for P. multocida prepared by:
providing a colony of P. multocida cells;
culturing the colony of cells under iron-restricted culture conditions in a suitable culture medium; harvesting the antigen from the culture at a suitable stage;
separating solids, including any of said cells from the resulting supernatant liquid so as to obtain a P. multocida, cell-free solution of the antigen which is essentially endotoxin-free; and
a pharmaceutically acceptable carrier or diluent.

35. A vaccine according to claim 34 wherein said supernatant is lyophilized or otherwise suitably concentrated for use.

36. A vaccine according to claim 34 or claim 35 wherein said carrier includes a pharmacologically expedient adjuvant.

37. A vaccine according to any one of claims 32 to 36 in combination with a protective amount of an antigen specific for P. haemolytica.

38. The use of a protective amount of the vaccine of claim 34 to vaccinate animals against pneumonic pasteurellosis.

39. A cell-free vaccine effective against pneumonic pasteurellosis in animals comprising:
a protective amount of a non-toxic inactive antigen specific for P. multocida prepared by:
culturing an innoculum of P. multocida having an optical density of about 0.01 to 0.03 measured at a wavelength of 525 nm in an iron-restricted medium for a period in the range of 3 to 3.5 hrs so as to produce said antigen;
periodically measuring the optical density of the medium;
upon detecting a value for the optical density of about 0.28 to 0.32, measured at a wavelength of 525 nm which indicates the phase of logarithmic growth of the cells when an optimum concentration of antigen is produced in said medium, separating supernatant liquid containing said cytotoxin from the resulting culture;
separating solids including any of said cells from the resulting supernatant liquid so as to obtain P. multocida cell-free solution of said antigen which is essentially endotoxin-free; and
a pharmaceutically acceptable carrier or diluent.

40. The use of a protective amount of the cell-free vaccine of claim 39 to vaccinate animals in need against pneumonic pasteurellosis.

41. A kit including an antigen or fragment thereof, against P. multocida prepared by the method according to any one of claims 1 to 17.

42. A diagnostic assay kit including an antigen, or fragment thereof, against P. multocida prepared by the method according to any one of claims 1 to 17.

43. A method of monitoring culture conditions in a culture of P. multocida including:
obtaining a sample of the culture of P. multocida ; and
monitoring an antigen of P. multocida.

44. A method according to claim 43 wherein the monitoring of the antigen is by detection by an antibody according to claim 22, a monoclonal antibody according to claim 24 or an antibody probe prepared by CAB technology.

45. A method according to claim 43 or claim 44 wherein the antigen is an antigen according to claim 19.

46. A method of monitoring culture conditions including:
measuring the level of at least one antigen of P. multocida and comparing the level with the level of at least one other antigen of P. multocida under similar culture conditions.

47. A method of monitoring antigen production in a culture of P. multocida including:
obtaining a sample of the culture of P. multocida; and monitoring the culture conditions.

48. A method of monitoring antigen production in a culture of P. multocida wherein the level of Iron Restriction in the culture is monitored.

49. A method of monitoring antigen production in a culture of P. multocida including measuring the production of at least one antigen of P. multocida and comparing the production of that antigen to deduce antigen production of another antigen produced under similar culture conditions.
